Europäisches Patentamt

European Patent Office

Office européen des brevets

(11) Publication number: **0 460 905 B1**

# EUROPEAN PATENT SPECIFICATION

(45) Date of publication of patent specification: **27.09.95**    (51) Int. Cl.6: **C07C 51/12**

(21) Application number: **91305019.1**

(22) Date of filing: **03.06.91**

(54) **Improved method for producing ibuprofen.**

(30) Priority: **04.06.90 US 533630**

(43) Date of publication of application:
**11.12.91 Bulletin 91/50**

(45) Publication of the grant of the patent:
**27.09.95 Bulletin 95/39**

(84) Designated Contracting States:
**AT BE CH DE ES FR GB IT LI NL SE**

(56) References cited:
**EP-A- 0 284 310**
**EP-A- 0 337 803**

(73) Proprietor: **HOECHST CELANESE CORPORA-TION**
**Route 202-206 North**
**Somerville, N.J. 08876 (US)**

(72) Inventor: **Hendricks, Joel D.**
**14505 Sapphire Lane,**
**Pineville**
**North Carolina 28134 (US)**
Inventor: **Mott, Graham N.**
**7417 Lake Como**
**Corpus Christi,**
**Texas 78413 (US)**

(74) Representative: **De Minvielle-Devaux, Ian Benedict Peter et al**
**CARPMAELS & RANSFORD**
**43, Bloomsbury Square**
**London WC1A 2RA (GB)**

**Description**

BACKGROUND OF THE INVENTION

Field of the Invention

This invention relates to an improved method for the production of 2-(4'-isobutylphenyl)propionic acid, more commonly known as ibuprofen.

Description of Related Art

The following information is disclosed in accordance with the terms of 37 CFR 1.56, 1.97 and 1.98.

Ibuprofen is a well-known nonsteroidal anti-inflammatory drug which has been converted from ethical, i.e., prescription, to over-the-counter status.

Japanese Kokai Patent No. SHO 55 [1980]-27,147, published Feb. 27, 1980 and assigned to Mitsubishi Petrochemical Co., discloses the formation of aryl substituted carboxylic acids, e.g., $\alpha$-(4'-isobutylphenyl)-propionic acid or ibuprofen, by reacting an aryl-substituted alcohol, e.g., 1-(4-isobutylphenyl)-ethanol, with carbon monoxide and water in the presence of a hydrogen fluoride catalyst.

Japanese Kokai Patent No. SHO 59 [1984]-95,238, published June 1, 1984 and assigned to Mitsubishi Petrochemical Co., teaches the formation of phenylacetic acid derivatives such as $\alpha$-aryl-substituted propionic acids, where the aryl group may be a phenyl group containing at least one alkoxy, aryloxy, hydroxy, or amino group as an electron-donor substituent, by reacting a benzyl alcohol derivative, which may be an $\alpha$-aryl substituted ethanol wherein the aryl group is the same as in the phenylacetic acid derivative product, with carbon monoxide and water, alcohol, or phenol, in the presence of a palladium catalyst. An acidic compound such as hydrogen chloride may be added as an auxiliary catalyst and a solvent such as benzene may also be used. The disclosure includes a comparative example in which ibuprofen (not included within the invention) is obtained in very low yield, i.e., 17.1%, when made utilizing the described process.

JP-A-59095239, published June 1, 1984 and assigned to Mitsubishi Petrochemical Co., discloses the formation of $\alpha$-(6-methoxy-2-naphthyl)propionic acid by reacting $\alpha$-(6-methoxy-2-naphthyl)ethyl alcohol with carbon monoxide and water in the presence of a palladium catalyst and an acidic compound, e.g., hydrogen chloride. The patent publication also states that if a non-halogen-containing acidic compound is used, it is desirable to add an ionizable metal halide to the reaction.

JP-B-56035659 published August 19, 1981 and assigned to Ferrer Int. SA, discloses an anhydrous method of producing a 2-(4'-isobutylphenyl)propionic acid ester by treating 1-(4'-isobutylphenyl)ethanol (IBPE) with carbon monoxide in a solution containing an alkanol and a catalyst such as palladium bis-(triphenylphosphine) dichloro complex. The solution may also contain up to 10% of a mineral acid such as hydrogen chloride.

Our EP-A-284310 published September 28, 1988 (based on USSN 28514 filed March 20, 1987 and USSN 158141 filed March 4, 1988) provides a method for the preparation of ibuprofen by carbonylating 1-(4'-isobutylphenyl)ethanol (IBPE) with carbon monoxide in an acidic aqueous medium, e.g. containing at least 10% of water based on the weight of IBPE initially added, at a temperature of at least 10°C and a carbon monoxide pressure of at least 500 psig, and in the presence of (1) a catalyst consisting essentially of a palladium compound in which the palladium has a valence of zero to 2 and is complexed with at least one monodentate phosphine ligand miscible with the organic phase of the reaction medium, the phosphorus/palladium mole ratio in said palladium compound and ligand being at least 2:1 when the palladium/IBPE mole ratio is below 1:10,000; (2) dissociated hydrogen ions from an acid which is substantially completely ionizable in dilute aqueous solution such that the mole ratio of hydrogen ions to IBPE added to the reaction zone is at least 0.15, and the mole ratio of hydrogen ions to water is at least 0.026; and (3) dissociated halide ions such that the mole ratio of halide ions to IBPE added to the reaction zone is at least 0.15. Advantageously, a hydrogen halide is the source.

Our EP-A-400892 published December 5, 1990 (based on USSN 357381 filed May 24, 1989 and USSN 500645 filed March 28, 1990) provides a method for the preparation of ibuprofen by carbonylating 1-(4'-isobutylphenyl)ethanol (IBPE) with carbon monoxide in an acidic aqueous medium e.g. containing at least 10% of water based on the weight of IBPE initially added, at a temperature of at least 10°C and a carbon monoxide pressure of at least 500 psig, and in the presence of (1) a catalyst complex consisting essentially of a palladium compound in which the palladium has a valence of zero to 2 and is complexed with at least one monodentate phosphine ligand miscible with the organic phase of the reaction medium, the phosphorus/palladium mole ratio in said palladium compound and ligand being at least 2:1 when the palladium/IBPE mole ratio is such that palladium = 1 and IBPE = 10,000 or more; (2) dissociated hydrogen ions from an acid which is substantially completely ionizable in dilute aqueous solution such that the mole ratio of hydrogen ions to IBPE added to the reaction zone is at least 0.15; and, (3) dissociated halide ions such that the mole ratio of halide ions to IBPE added to the reaction zone is at least 0.15. Advantageously, a hydrogen halide is the source of hydrogen ions and halide ions. The carbonylation is preferably integrated with a method of producing IBPE from isobutylbenzene wherein the latter compound is subjected to Friedel-Crafts reaction with an acetylating agent to produce 4-isobutylacetophenone, which is then reduced with hydrogen in the presence of a hydrogenation catalyst, or with a reducing agent containing available hydrogen, to obtain IBPE.

The palladium catalyst complex can be precipitated from an organic phase of the carbonylation reaction which typically includes the ibuprofen product. The precipitated catalyst complex can be recycled without further treatment.

It has surprisingly been found that the inclusion of some ibuprofen in the initial reaction medium substantially accelerates the overall reaction and thus results in a significant and valuable reduction of the time needed to produce a given yield of ibuprofen.

In one aspect, the invention relates to the use of ibuprofen to accelerate the reaction in a process for the production of ibuprofen by reaction of IBPE and carbon monoxide in the presence of a carbonylation catalyst.

In another aspect, the invention relates to a process for the production of 2-(4'-isobutylphenyl)propionic acid, i.e. ibuprofen, by the carbonylation of 1-(4'-isobutyl-phenyl)ethanol (IBPE) comprising initially contacting carbon monoxide with a reaction mixture containing IBPE, ibuprofen and a catalyst for the carbonylation under conditions to initiate the reaction, and continuing to feed carbon monoxide to the composition to produce the desired amount of ibuprofen.

It has been found that the method of this invention, including the presence of ibuprofen in the initial feed composition, has a significant effect in reducing the amount of reaction time necessary to obtain a major proportion of the possible yield of ibuprofen.

DESCRIPTION OF PREFERRED EMBODIMENTS

The method of the invention is capable of yielding an advantage in reaction time using operable carbonylation catalyst and reaction conditions in an aqueous medium. This improvement in reaction time makes possible the use of continuous reaction processes in addition to the batch processes previously developed. Preferably, the reaction is carried out in an acidic aqueous medium using a catalyst comprising palladium and a monodentate phosphine ligand, and in the case of a batch reaction, or a continuous plug flow reaction, the operable conditions used are typically those disclosed in our previously cited EP-A-400892. These conditions include carrying out a batch-type reaction in an acidic aqueous medium at a temperature of at least 10°C and a carbon monoxide pressure of at least 500 psig, and in the presence of 1) a catalyst consisting essentially of a palladium compound in which the palladium has a valence of zero to 2 and is complexed with at least one acid stable, monodentate phosphine ligand freely miscible with the organic phase of the reaction medium, the phosphorus/palladium mole ratio in said palladium compound and ligand being at least 2:1 when the mole ratio of palladium to IBPE is such that palladium = 1 and IBPE = 10,000 or more; 2) ionizable or dissociatable hydrogen ions in the form of an acid which is substantially ionizable in a dilute aqueous solution, e.g. of 0.1N concentration, such that the mole ratio of hydrogen ions to IBPE added to the reaction zone (H+/IBPE) is at least 0.15; and 3) ionizable or dissociatable halide ions such that the mole ratio of halide ions to IBPE added to the reaction zone (X⁻/IBPE) is at least 0.15.

When the mole ratio of palladium to IBPE in the above-described reaction is such that palladium = 1 and IBPE is less than 10,000 the phosphorus to palladium mole ratio in said palladium compound and ligand typically ranges from 0.1 to 50, although the maximum mole ratio of phosphorus to palladium does not appear to be critical.

The term "monodentate" is intended to mean a single phosphine phosphorus atom present in the ligand molecule before it is complexed with palladium. The phrase "freely miscible with the organic phase of the reaction medium" means that the ligand is not complexed with an insoluble substrate such as a polymer which prevents it from being freely mixed in the organic phase.

In carrying out a batch or continuous plug flow carbonylation reaction, the mole ratio of ibuprofen to IBPE at the initiation of the reaction, i.e., when the reaction composition is initially contacted with carbon monoxide under reaction conditions, may be in the range of 0.01 to 2, preferably 0.1 to 1.25; water may be present in an amount, for example, of 10 to 600%, preferably 15 to 100%, based on the weight of IBPE initially present; the temperature of reaction may be, for example, in the range of 10 to 225°C, preferably 70 to 175°C; the carbon monoxide pressure may be, for example, in the range of 500 to 5000 psig, preferably 700 to 3000 psig; and the total reaction time may be, for example, in the range of 0.25 to 2.0 hours, preferably 0.25 to 0.75 hour. This compares with typical total reaction time periods of 2 hours to 2.5 hours to obtain an equivalent product yield when ibuprofen is not present at the initiation of the reaction.

In carrying out a continuous carbonylation reaction where backmixing occurs, the required mole ratio of ibuprofen to IBPE in the feed to the reaction system is expected to be higher, for example, in the range of 15 to 1,000. This mole ratio requirement is affected by the concentration of reactants in the reaction system and by the concentration of ibuprofen required in the product exiting the reaction system. The preferred mole ratio of ibuprofen to IBPE in the feed to a continuous carbonylation reaction wherein substantial backmixing occurs is expected to range from 20 to 200.

Some palladium catalysts which may be used wherein the palladium is complexed with an appropriate ligand are as follows: bis(triphenylphosphine) dichloro complex, bis(tributylphosphine) dichloro complex, bis-(tricyclohexylphosphine) dichloro complex, pi-allyltriphenylphosphine dichloro complex, triphenylphosphine piperidine dichloro complex, bis(triphenylphosphine) dicarbonyl complex, bis(triphenylphosphine) diacetate complex, bis(triphenylphosphine) dinitrate complex, bis(triphenylphosphine) sulfate complex, tetrakis-(triphenylphosphine) complex, and complexes in which some of the ligands are carbon monoxide such as chlorocarbonyl bis(triphenylphosphine) complex, all complexes of palladium. Also suitable as a catalyst is palladium metal on a suitable catalyst support such as carbon, alumina, silica, or an inert polymer which can tolerate the conditions of reaction, complexed with one or more of these foregoing ligands.

The palladium salts and phosphine ligands making up the foregoing catalyst complexes may also be added separately to the reaction zone. In this case, the amount of ligand added is preferably sufficient to complex with the palladium present such that the P:Pd mole ratio is equal to at least 1:1 when the Pd:IBPE mole ratio is at least 1:5,000. However, when the latter ratio is below 1:10,000, it is necessary to use an excess of phosphine ligand such that the P:Pd ratio is at least 2:1.

The catalyst complex may be present in an amount such that the mole ratio of palladium to IBPE is in the range, for example, of 1:25 to 1:60,000, preferably 1:150 to 1:50,000.

The ionizable or dissociatable hydrogen ions and halide ions may be conveniently added to the reaction as hydrogen chloride, hydrogen bromide, or hydrogen iodide. However, it is also possible to add the hydrogen ions and halide ions from separate sources. For example, other acids completely ionizable in dilute aqueous solution, e.g., inorganic acids, such as sulfuric acid, phosphoric acid or polyphosphoric acid, or organic acids, e.g., sulfonic acids such as p-toluenesulfonic acid, methanesulfonic acid, or trifluoroacetic acid, may be used as the source of hydrogen ions. Similarly, other water-soluble and ionizable halide compounds, as for example, halide salts wherein the cation does not interfere with the reaction, e.g., alkali metal halides such as potassium, sodium, and lithium chlorides, bromides, and iodides may be used as the source of halide ions. The mole ratio of hydrogen ions and halide ions to IBPE ($H^+$/IBPE and $X^-$/IBPE) each may be in the range, for example, of 0.15 to 5, preferably 0.3 to 2.0.

Although not necessary for the operability of the process, in some instances, it may be advantageous to utilize an organic solvent for the reaction. Organic solvents which can be used are, for example, ketones such as methyl ethyl ketone, acetone, 2-pentanone, 3-pentanone, and acetophenone, aromatic hydrocarbons such as benzene and toluene, and cyclic ethers such as tetrahydrofuran and dioxane. Ketones and ethers are preferred if a solvent is used. If the catalytic palladium as added to the system is in the metallic or zero valence state (Pd°), then any solvent used should be non-hydrocarbon. The solvent may be present in a weight ratio of solvent to IBPE in the range, for example, of 0 to 1000:1, preferably 0 to 10:1.

An inorganic salt may also be present during the reaction. Inorganic salts which may be used are, for example, those yielding anions comprising oxygen, and sulfur, phosphorus, aluminum, or silicon, including such anions as hydrogensulfate, pyrosulfate, ortho-phosphate, pyrophosphate, aluminate, or silicate and cations such as sodium, potassium, calcium, or magnesium, or another cation which does not interfere with the reaction, e.g., ammonium or alkylammonium such as tetrabutylammonium. Other inorganic salts such as calcium chloride may also be added. The inorganic salt, if used, will generally be present at a concentration of, for example, 0.1 to 50%, preferably 1 to 20% by weight of total charge.

In some instances, an undesirable heavy ends fraction may form during the reaction, possibly due to a polymerization mechanism of unknown nature. In view of this, it may be beneficial to incorporate a polymerization inhibitor in the reaction mass. Inhibitors which may be used for this purpose include, for example, t-butylcatechol, hydroquinone, m-dinitrobenzene, N-nitrosodiphenylamine, picric acid, sodium sulfite, quinhydrone and the like. If an inhibitor is utilized, it may be incorporated in an amount, for example, of 0.01 to 15%, preferably 0.1 to 5% by weight based on the weight of IBPE.

As stated, the inventive method makes it possible to obtain a major proportion of the desired yield of ibuprofen in substantially less time than is the case when the initial reaction mixture containing IBPE does not also contain ibuprofen. This result can be exploited whether the method is carried out batchwise, semi-continuously or continuously. In batch operation, the ibuprofen is added to the reaction with the other components of the reaction mixture before the reaction is initiated with CO at the reaction temperature. After the conclusion of the reaction which requires a much shorter time (the reaction time can be, typically, one quarter of the reaction time required when no ibuprofen is added initially), the CO flow is cut off, the contents of the reactor are removed for purification, and the procedure is repeated with the next batch. In semicontinuous operation, the initial procedure is similar to batch operation. However, after the reaction is substantially concluded and the CO flow is cut off and the reaction vented, not all the contents of the reactor are removed for purification. Rather, a small amount of these contents, which contain a large proportion of ibuprofen, may be allowed to remain in the reactor to serve as a "heel", under the invention. Fresh amounts of the other components of the reaction mixture may then be added, including IBPE aqueous acid, and catalyst, and the reaction again initiated by contacting the mixture with CO and reestablishing reaction temperature. This procedure may then be repeated as many times as convenient. In continuous operation, if little or no backmixing occurs in the reactor as in the case of certain tubular reactors, the advantages of the inventive method may be obtained by continuously feeding ibuprofen into the reactor with the other components of the charge composition. This may be accomplished by recycling a small proportion of the product stream to the charge end of the reactor. If substantial backmixing occurs in the continuous reactor, then an advantage of the inventive method in terms of an increase in the initial reaction rate may be obtained by adding ibuprofen to the reactor with the other components of the feed so that such ibuprofen is present when CO feed is initiated.

Examples 1 to 20 further illustrate the invention. In these examples, the "time of reaction" is the actual time that it takes for the reaction to proceed to substantial completeness as indicated by CO uptake. Such time, which may be also termed as the "period of actual reaction", is measured from the onset of the reaction at about 130°C, when the CO uptake ascribed to the reaction becomes significant, to the point at which such CO uptake is at or close to zero. Such measured time is different from the "time of reaction" given in the examples of previously cited EP-A-400892 and its predecessor, which was a previously set time from the onset of the reaction at about 130°C to the point at which the reactor contents were cooled below the necessary reaction temperature prior to removal for subsequent handling. Although the latter preset time is generally estimated to be approximately equal to the period of actual reaction measured by CO uptake as previously defined, it could be somewhat higher or lower than such period.

### Examples 1 to 20

To a 4 liter Hastalloy B autoclave were added varying amounts of IBPE, 36% or 26% aqueous hydrochloric acid, palladium chloride, triphenyl phosphine, and ibuprofen. The autoclave was sealed, purged with $N_2$ and CO, and pressured with CO to a level sufficient to result in a targeted reaction pressure at a reaction temperature of 130°C or 140°C. The reactor contents were then heated to the latter reaction temperature and CO was fed to the reactor to maintain the desired pressure as the CO was absorbed during the reaction. Substantial completion of the reaction was determined by monitoring the CO uptake. In each case, conversion of IBPE was at least 99%. The conditions of reaction including quantities of reaction components (wherein ibuprofen is indicated as "IBU"), mole ratio of hydrogen ions to IBPE ($H^+$/IBPE) mole ratio of ibuprofen to IBPE (IBU/IBPE), temperature, time of reaction (i.e., the period of actual reaction measured by CO uptake as defined previously), pressure, and results of the reaction in terms of ibuprofen selectivity (IBU Sel.), are shown in the following table.

TABLE

| Example | IBPE, mmol | HCl, %/mL | H+/IBPE | PdCl₂, mmol | PPh₃, mmol | IBU, mmol | IBU/IBPE | Temp. °C | Time, min. | Press., psig | IBU/Sel. %( 2%) |
|---|---|---|---|---|---|---|---|---|---|---|---|
| 1 | 5899 | 26/541 | 0.75 | 2.0 | 5.0 | 1969 | 0.33 | 130 | 90 | 1000 | 81 |
| 2 | 8427 | 26/775 | 0.75 | 2.8 | 6.9 | 2809 | 0.33 | 130 | 80-90 | 1500 | 90 |
| 3 | 8427 | 26/775 | 0.75 | 2.8 | 6.9 | 2809 | 0.33 | 130 | 70 | 2400 | 91 |
| 4 | 8427 | 26/775 | 0.75 | 2.8 | 6.9 | 2809 | 0.33 | 130 | 100 | 1500 | 90 |
| 5 | 5899 | 36/371 | 0.75 | 2.0 | 5.0 | 1967 | 0.33 | 130 | 70 | 2400 | 81 |
| 6 | 5899 | 36/371 | 0.75 | 2.0 | 5.0 | 1967 | 0.33 | 130 | 50-60 | 2400 | 90 |
| 7 | 5899 | 36/621 | 1.26 | 2.0 | 5.0 | 1967 | 0.33 | 140 | 80 | 1000 | 78 |
| 8 | 5899 | 26/906 | 1.26 | 2.0 | 5.0 | 1967 | 0.33 | 140 | — | 2400 | 83 |
| 9 | 8427 | 26/541 | 0.75 | 2.8 | 6.9 | 3933 | 0.67 | 130 | 80 | 1500 | 85 |
| 10 | 5899 | 26/541 | 0.75 | 2.0 | 5.0 | 5618 | 0.67 | 140 | 90 | 2400 | 88 |
| 11 | 5899 | 36/371 | 0.75 | 2.0 | 5.0 | 5900 | 1.00 | 140 | 90 | 1000 | 82 |
| 12 | 5899 | 36/371 | 0.75 | 2.0 | 5.0 | 5900 | 1.00 | 140 | 30-40 | 2400 | 88 |
| 13 | 5618 | 26/769 | 1.11 | 2.8 | 6.9 | 5632 | 1.00 | 130 | 40-50 | 2400 | 94 |
| 14 | 5618 | 26/775 | 1.13 | 2.0 | 5.0 | 5632 | 1.00 | 130 | 50-60 | 2400 | 90 |
| 15 | 5899 | 36/621 | 1.26 | 2.0 | 5.0 | 5900 | 1.00 | 130 | 40 | 2400 | 85 |
| 16 | 5899 | 36/621 | 1.26 | 2.0 | 5.0 | 5900 | 1.00 | 130 | 80-90 | 1000 | 75 |
| 17 | 5899 | 36/621 | 1.26 | 2.0 | 5.0 | 5900 | 1.00 | 130 | 60 | 1000 | 54 |
| 18 | 5899 | 36/906 | 1.26 | 2.0 | 5.0 | 5900 | 1.00 | 130 | 100 | 1000 | 80 |
| 19 | 5899 | 26/906 | 1.26 | 2.0 | 5.0 | 5900 | 1.00 | 130 | 40-50 | 2400 | 83 |
| 20 | 5899 | 26/907 | 1.26 | 2.0 | 5.0 | 5900 | 1.00 | 130 | 30-40 | 2400 | 92 |

Comparative Example A

The procedure of Example 3 was followed utilizing 26% HCl except that no ibuprofen was added with the initial feed components. The period of actual reaction as determined by CO uptake was about 120

6

EP 0 460 905 B1

minutes, the IBPE conversion was over 99% and the selectivity to ibuprofen ranged from about 93 to 95%.

Comparative Example B

The procedure of Example 6 was followed utilizing 36% HCl, except that no ibuprofen was added with the initial feed components. The period of actual reaction as determined by CO uptake was about 130 minutes, the IBPE conversion was over 99% and the selectivity to ibuprofen ranged from about 93 to 95%.

A comparison of the results of Examples 3 and 6 with those of Comparative Examples A and B, respectively, indicate that the presence of ibuprofen in the feed composition when the reaction is initiated with CO make it possible to obtain high selectivities of ibuprofen with substantially complete conversion of IBPE at much shorter reaction times than if no ibuprofen is present.

**Claims**

1. A method for production of ibuprofen by the carbonylation of 1'(4'-isobutylphenyl)ethanol (IBPE) comprising initially contacting a reaction mixture comprising IBPE, ibuprofen and a catalyst for the carbonylation with carbon monoxide under conditions to initiate reaction, and continuing to feed carbon monoxide to the composition to produce the desired yield of ibuprofen.

2. The method of claim 1 wherein the reaction is a batch reaction or a continuous plug flow reaction and the mole ratio of ibuprofen to IBPE at the initiation of the reaction with carbon monoxide is in the range of 0.01 to 2.

3. The method of claim 2 wherein the mole ratio of ibuprofen to IBPE at the initiation of the reaction with carbon monoxide is in the range of 0.1 to 1.25.

4. The method of any of claims 1-3 wherein the reaction is a batch reaction or a continuous plug flow reaction and the carbonylation is carried out in an acidic aqueous medium, at a temperature of at least 10°C and a carbon monoxide pressure of at least 500 psig, and in the presence of (1) a palladium catalyst in which the palladium has a valence of zero to 2 and is complexed with at least one acid stable, monodentate phosphine ligand miscible with the organic phase of the reaction medium, the phosphorus/palladium mole ratio in said palladium compound and ligand being at least 2:1 when the mole ratio of palladium to IBPE is such that palladium = 1 and IBPE = 10,000 or more; (2) ionizable or dissociatable hydrogen ions in the form of an acid which is ionizable in a dilute aqueous solution such that the mole ratio of hydrogen ions to IBPE added to the reaction zone is at least 0.15; (3) ionizable or dissociatable halide ions such that the mole ratio of halide ions to IBPE added to the reaction zone is at least 0.15.

5. The method of claim 4 wherein the source of said hydrogen ions and halide ions is a hydrogen halide.

6. The method of claim 5 wherein said hydrogen halide is hydrogen chloride.

7. The method of claim 1 wherein the reaction is a continuous reaction where backmixing occurs, and wherein the mole ratio of ibuprofen to IBPE at the initiation of the reaction with carbon monoxide is in the range of 15 to 1,000.

8. The method of claim 7 wherein the mole ratio of ibuprofen to IBPE ranges from 20 to 200.

9. The method of any of claims 1-8 wherein the catalyst comprises palladium and a monodentate phosphine ligand.

10. The method of claim 9 wherein the palladium is added in the form of palladium chloride.

11. The method of claim 9 or 10 wherein the ligand is a tri(organo) phosphine.

12. The method of claim 11 wherein the ligand is triphenyl phosphine.

7

13. Use of ibuprofen in the initial reaction mixture to accelerate the reaction in a process for the production of ibuprofen by reaction of 1-(4'-isobutylphenyl)ethanol and carbon monoxide in the presence of a carbonylation catalyst.

**Patentansprüche**

1. Verfahren zur Herstellung von Ibuprofen durch Carbonylierung von 1-(4'-Isobutylphenyl)ethanol (IBPE), umfassend das anfängliche In-Kontakt-Bringen eines Reaktionsgemischs, das IBPE, Ibuprofen und einen Katalysator für die Carbonylierung umfaßt, mit Kohlenmonoxid unter Bedingungen, bei denen die Reaktion gestartet wird, und das weitere Zuführen von Kohlenmonoxid zu der Zusammensetzung, so daß die gewünschte Ausbeute an Ibuprofen gebildet wird.

2. Verfahren gemäß Anspruch 1, wobei die Reaktion eine Chargenreaktion oder eine kontinuierliche Pfropfenströmungsreaktion ist und das Stoffmengenverhältnis von Ibuprofen zu IBPE beim Start der Reaktion mit Kohlenmonoxid im Bereich von 0,01 bis 2 liegt.

3. Verfahren gemäß Anspruch 2, wobei das Stoffmengenverhältnis von Ibuprofen zu IBPE beim Start der Reaktion mit Kohlenmonoxid im Bereich von 0,1 bis 1,25 liegt.

4. Verfahren gemäß einem der Ansprüche 1-3, wobei die Reaktion eine Chargenreaktion oder eine kontinuierliche Pfropfenströmungsreaktion ist und die Carbonylierung durchgeführt wird in einem sauren wäßrigen Medium bei einer Temperatur von wenigstens 10°C und einem Kohlenmonoxiddruck von wenigstens 500 psig und in Gegenwart von (1) einem Palladiumkatalysator, in dem das Palladium eine Wertigkeit von 0 bis 2 hat und mit wenigstens einem säurestabilen einzähnigen Phosphinliganden, der mit der organischen Phase des Reaktionsmediums mischbar ist, komplexiert ist, wobei das Stoffmengenverhältnis Phosphor/Palladium in der Palladiumverbindung und dem Liganden wenigstens 2:1 beträgt, wenn das Stoffmengenverhältnis von Palladium zu IBPE so ist, daß Palladium = 1 und IBPE = 10000 oder mehr ist, (2) ionisierbaren oder dissoziierbaren Wasserstoffionen in Form einer Säure, die in einer verdünnten wäßrigen Lösung ionisierbar ist, so daß das Stoffmengenverhältnis von Wasserstoffionen zu IBPE, das in die Reaktionszone gegeben wird, wenigstens 0,15 beträgt, (3) ionisierbaren oder dissoziierbaren Halogenidionen, so daß das Stoffmengenverhältnis von Halogenidionen zu IBPE, das in die Reaktionszone gegeben wird, wenigstens 0,15 beträgt.

5. Verfahren gemäß Anspruch 4, wobei die Quelle der Wasserstoffionen und Halogenidionen ein Halogenwasserstoff ist.

6. Verfahren gemäß Anspruch 5, wobei es sich bei dem Halogenwasserstoff um Chlorwasserstoff handelt.

7. Verfahren gemäß Anspruch 1, wobei die Reaktion eine kontinuierliche Reaktion ist, bei der ein Rückmischen erfolgt, und wobei das Stoffmengenverhältnis von Ibuprofen zu IBPE beim Start der Reaktion mit Kohlenmonoxid im Bereich von 15 bis 1000 liegt.

8. Verfahren gemäß Anspruch 7, wobei das Stoffmengenverhältnis von Ibuprofen zu IBPE im Bereich von 20 bis 200 liegt.

9. Verfahren gemäß einem der Ansprüche 1-8, wobei der Katalysator Palladium und einen einzähnigen Phosphinliganden umfaßt.

10. Verfahren gemäß Anspruch 9, wobei das Palladium in Form von Palladiumchlorid hinzugefügt wird.

11. Verfahren gemäß Anspruch 9 oder 10, wobei der Ligand ein Triorganophosphin ist.

12. Verfahren gemäß Anspruch 11, wobei es sich bei dem Liganden um Triphenylphosphin handelt.

13. Verwendung von Ibuprofen in dem anfänglichen Reaktionsgemisch, um in einem Verfahren zur Herstellung von Ibuprofen durch Reaktion von 1-(4'-Isobutylphenyl)ethanol und Kohlenmonoxid in Gegenwart eines Carbonylierungskatalysators die Reaktion zu beschleunigen.

EP 0 460 905 B1

**Revendications**

1. Procédé de production d'ibuprofène par carbonylation du 1-(4'-isobutylphényl)éthanol (IBPE), comprenant initialement la mise en contact d'un mélange réactionnel comprenant du IBPE, de l'ibuprofène et un catalyseur de carbonylation avec du monoxyde de carbone dans des conditions déclenchant la réaction, et le maintien du contact du monoxyde de carbone avec la composition pour obtenir le rendement désiré en ibuprofène.

2. Procédé suivant la revendication 1, dans lequel la réaction est une réaction discontinue ou une réaction continue à écoulement bouchon, et le rapport molaire de l'ibuprofène au IBPE au début de la réaction avec le monoxyde de carbone est compris dans l'intervalle de 0,01 à 2.

3. Procédé suivant la revendication 2, dans lequel le rapport molaire de l'ibuprofène au IBPE au début de la réaction avec le monoxyde de carbone est compris dans l'intervalle de 0,1 à 1,25.

4. Procédé suivant l'une quelconque des revendications 1 à 3, dans lequel la réaction est une réaction discontinue ou une réaction continue à écoulement bouchon, et la carbonylation est effectuée dans un milieu aqueux acide, à une température d'au moins 10°C et sous une pression de monoxyde de carbone d'au moins 500 psig, et en présence (1) d'un catalyseur au palladium dans lequel le palladium a une valence de zéro à 2 et est complexé avec au moins un ligand phosphine monodenté, stable en milieu acide, miscible à la phase organique du milieu réactionnel, le rapport molaire phosphore/palladium dans ledit composé de palladium et ledit ligand étant au moins égal à 2:1 lorsque le rapport molaire du palladium au IBPE est tel que palladium = 1 et IBPE = 10 000 ou plus ; (2) d'ions hydrogène ionisables ou dissociables, sous forme d'un acide qui est ionisable dans une solution aqueuse diluée de telle sorte que le rapport molaire des ions hydrogène au IBPE introduit dans la zone réactionnelle soit au moins égal à 0,15 ; (3) d'ions halogénure ionisables ou dissociables, de telle sorte que le rapport molaire des ions halogénure au IBPE introduit dans la zone réactionnelle soit au moins égal à 0,15.

5. Procédé suivant la revendication 4, dans lequel la source des ions hydrogène et des ions halogénure consiste en un halogénure d'hydrogène.

6. Procédé suivant la revendication 5, dans lequel l'halogénure d'hydrogène est le chlorure d'hydrogène.

7. Procédé suivant la revendication 1, dans lequel la réaction est une réaction continue dans laquelle se produit un mélange à contre-courant, et dans lequel le rapport molaire de l'ibuprofène au IBPE au début de la réaction avec le monoxyde de carbone est compris dans l'intervalle de 15 à 1000.

8. Procédé suivant la revendication 7, dans lequel le rapport molaire de l'ibuprofène au IBPE est compris dans l'intervalle de 20 à 200.

9. Procédé suivant l'une quelconque des revendications 1 à 8, dans lequel le catalyseur comprend du palladium et un ligand phosphine monodenté.

10. Procédé suivant la revendication 9, dans lequel le palladium est ajouté sous forme de chlorure de palladium.

11. Procédé suivant la revendication 9 ou 10, dans lequel le ligand est une triorganophosphine.

12. Procédé suivant la revendication 11, dans lequel le ligand consiste en triphénylphosphine.

13. Utilisation d'ibuprofène dans le mélange réactionnel initial pour accélérer la réaction dans un procédé de production d'ibuprofène par réaction du 1-(4'-isobutylphényl)éthanol et du monoxyde de carbone en présence d'un catalyseur de carbonylation.

9